(19)

Europäisches
Patentamt
European
Patent Office
Office européen
des brevets

(11)  **EP 2 624 155 A2**

(12)  **EUROPEAN PATENT APPLICATION**

(43) Date of publication:
**07.08.2013 Bulletin 2013/32**

(51) Int Cl.:
***G06F 19/00*** (2011.01)

(21) Application number: **12161590.0**

(22) Date of filing: **27.03.2012**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME** | (72) Inventors:<br>• **Lee, Suk-jin**<br>  **250-875 Gangwon-do (KR)**<br>• **Kim, Sung-hee**<br>  **250-875 Gangwon-do (KR)**<br>• **Kim, Jung-yi**<br>  **250-875 Gangwon-do (KR)** |
| (30) Priority: **06.02.2012 KR 20120011794** | (74) Representative: **Schmid, Wolfgang**<br>**Lorenz & Kollegen** |
| (71) Applicant: **Samsung Medison Co., Ltd.**<br>**Gangwon-do 250-875 (KR)** | **Patentanwälte Partnerschaftsgesellschaft**<br>**Alte Ulmer Strasse 2**<br>**89522 Heidenheim (DE)** |

(54)  **Method and apparatus for providing ultrasound image data**

(57)   An ultrasound image data providing method, the method including: storing ultrasound image data including an ultrasound image and tag information relating to the ultrasound image; receiving a predetermined search word; extracting ultrasound image data including tag information relating to the predetermined search word; and displaying the extracted ultrasound image data.

EP 2 624 155 A2

## Description

<u>CROSS-REFERENCE TO RELATED PATENT APPLICATION</u>

This application claims the benefit of Korean Patent Application No. 10-2012-0011794, filed on February 6, 2012, in the Korean Intellectual Property Office, the disclosure of which is incorporated herein in its entirety by reference.

<u>BACKGROUND OF THE INVENTION</u>

1. Field of the Invention

The present invention relates to a method and apparatus for providing ultrasound image data including an ultrasound image and tag information.

2. Description of the Related Art

Ultrasound diagnosis apparatuses transmit an ultrasound signal toward a predetermined part inside a body from a surface of the body and obtain an image of a tomogram of soft-tissues or blood flow by using information regarding the ultrasound signal reflected by tissues of the body.

Ultrasound diagnosis apparatuses have various advantages, including a compact size, low cost, and real-time display. Also, ultrasound diagnosis apparatuses have excellent stability because there is no fear of X-ray exposure, and thus, the ultrasound diagnosis apparatuses are commonly used together with other diagnosis apparatuses, such as computerized tomography (CT) scanners, magnetic resonance imaging (MRI) apparatuses, nuclear medicine diagnosis apparatuses, or the like.

In general, ultrasound diagnosis apparatuses do not provide a function of searching for an ultrasound image. That is, when a user searches for an ultrasound image of a previously diagnosed patient, the user needs to search for the ultrasound image by checking diagnosis dates and thumbnail images one by one. Thus, it takes much time to search for the ultrasound image desired by the user.

Furthermore, current ultrasound diagnosis apparatuses do not search for a plurality of ultrasound images having common characteristics simultaneously, and do not provide users with comparison data of the plurality of ultrasound images.

<u>SUMMARY OF THE INTENTION</u>

The present invention provides a method and apparatus for providing ultrasound image data including an ultrasound image and tag information so that a user can reduce time taken to search for the ultrasound image data and efficiently utilize the ultrasound image data.

According to an aspect of the present invention, there is provided an ultrasound image data providing method, the method including: storing ultrasound image data including an ultrasound image and tag information relating to the ultrasound image; receiving a predetermined search word; extracting ultrasound image data including tag information relating to the predetermined search word; and displaying the extracted ultrasound image data.

The tag information may include at least one of measurement information, annotation information, indication information, and body mark information, and the predetermined search word may include at least one of text and an image.

The displaying may include: receiving selection information regarding at least one tag included in the displayed ultrasound image data; and displaying an ultrasound image corresponding to the at least one tag selected according to the selection information.

The displaying may include: receiving additional tag information relating to the displayed ultrasound image data from a user; and displaying the ultrasound image data including the received additional tag information.

The method may include: extracting a plurality of pieces of ultrasound image data including the tag information relating to the predetermined search word; displaying the extracted plurality of pieces of ultrasound image data; displaying a result obtained by comparing the extracted plurality of pieces of ultrasound image data; generating a comparison result as tag information; and storing ultrasound image data including the generated tag information.

According to another aspect of the present invention, there is provided an ultrasound image data providing method, the method including: acquiring an ultrasound image regarding a target; generating tag information relating to the acquired ultrasound image; and displaying ultrasound image data including the acquired ultrasound image and the generated tag information.

According to another aspect of the present invention, there is provided an ultrasound image data providing apparatus, the apparatus including: a storage unit for storing ultrasound image data including an ultrasound image and tag information relating to the ultrasound image; a receiving unit for receiving a predetermined search word; an extraction unit for extracting ultrasound image data including tag information relating to the predetermined search word; a displaying unit for displaying the extracted ultrasound image data; and a control unit for controlling the storage unit, the receiving unit, the extraction unit, and the display unit.

According to another aspect of the present invention, there is provided an ultrasound image data providing apparatus, the apparatus including: an acquisition unit for acquiring an ultrasound image regarding a target; a tag generation unit for generating tag information relating to the acquired ultrasound image; and a display unit for displaying ultrasound image data including the acquired ultrasound image and the generated tag information.

BRIEF DESCRIPTION OF THE DRAWINGS

The above and other features and advantages of the present invention will become more apparent by describing in detail exemplary embodiments thereof with reference to the attached drawings in which:

FIG. 1 is a block diagram of an ultrasound image data providing apparatus according to an embodiment of the present invention;
FIG. 2 is a flowchart illustrating an ultrasound image data providing method, according to an embodiment of the present invention;
FIG. 3 is a diagram for describing ultrasound image data including tag information, according to an embodiment of the present invention;
FIG. 4 is a diagram for describing a method of providing an ultrasound image corresponding to a specific tag selected by a user, according to an embodiment of the present invention;
FIG. 5 is a flowchart illustrating a method of providing a plurality of pieces of ultrasound image data, according to an embodiment of the present invention;
FIG. 6 is a diagram for describing a search result with respect to ultrasound image data, according to an embodiment of the present invention;
FIG. 7 is a diagram for describing a method of displaying a comparison result with respect to a plurality of pieces of ultrasound image data, according to an embodiment of the present invention; and
FIG. 8 is a flowchart illustrating a method of displaying ultrasound image data, according to an embodiment of the present invention.

DETAILED DESCRIPTION OF THE INVENTION

First, the terms used in the present disclosure will be briefly described below before exemplary embodiments of the present invention are described in greater detail.
Most of the terms used herein are general terms that have been widely used in the technical art to which the present invention pertains. However, some of the terms used herein may be created reflecting intentions of technicians in this art, precedents, or new technologies. Also, some of the terms used herein may be arbitrarily chosen by the present applicant. In this case, these terms are defined in detail below. Accordingly, the specific terms used herein should be understood based on the unique meanings thereof and the whole context of the present invention.
In the present specification, it should be understood that the terms, such as 'including' or 'having,' etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Also, the terms, such as 'unit' or 'module', etc., should be understood as a unit that processes at least one function or operation and that may be embodied in a hardware manner, a software manner, or a combination of the hardware manner and the software manner. Expressions such as "at least one of," when preceding a list of elements, modify the entire list of elements and do not modify the individual elements of the list.
Throughout the specification, the term 'ultrasound image' means an image of a target, which is captured using ultrasound waves. Here, the 'target' may be understood to be part of a human body. For example, the target may be an organ of a human body, such as the liver, the heart, the womb, a brain, the breast, or the abdomen, or a fetus.
Throughout the specification, the term 'user' may be a medical professional, e.g., a doctor, a nurse, a clinical pathologist, or a medical imaging professional, but is not limited thereto.

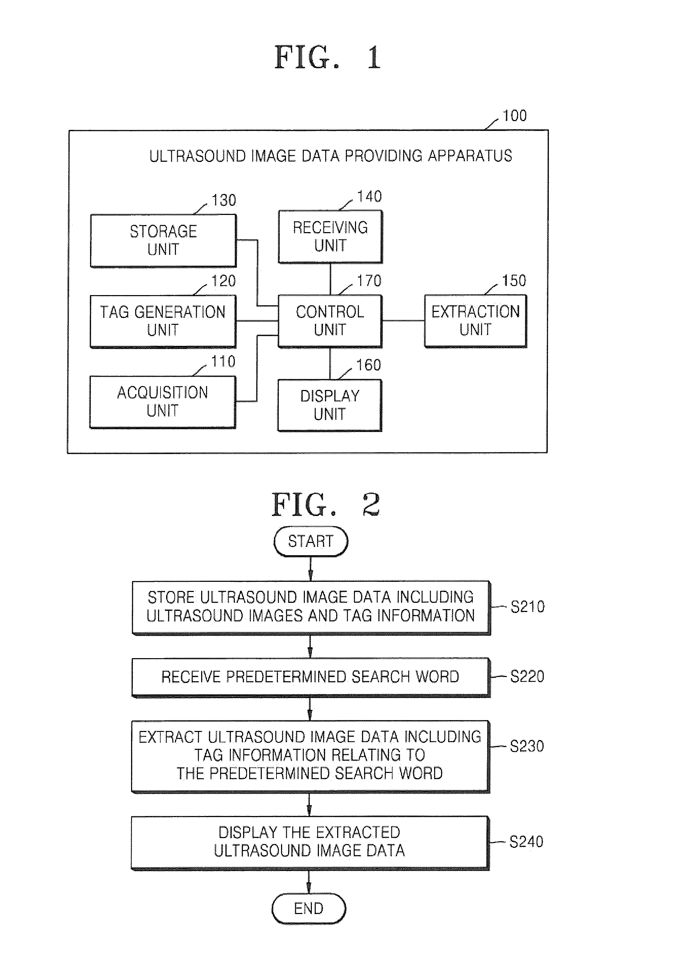

FIG. 1 is a block diagram of an ultrasound image data providing apparatus 100 according to an embodiment of the present invention.
According to the present embodiment, as shown in FIG. 1, the ultrasound image data providing apparatus 100 may include an acquisition unit 110, a tag generation unit 120, a storage unit 130, a receiving unit 140, an extraction unit 150, a display unit 160, and a control unit 170. However, the elements of FIG. 1 are not indispensable elements. The ultrasound image data providing apparatus 100 may include a greater number of elements or a lesser number of elements.
The acquisition unit 110 may acquire an ultrasound image of a target. For example, the acquisition unit 110 may receive an ultrasound echo signal corresponding to an ultrasound signal output by an ultrasound output unit and form the ultrasound image of the target.
According to an embodiment of the present invention, the ultrasound image may be a 2-dimensional (2D) image or a 3D image.
The tag generation unit 120 may generate tag information relating to the ultrasound image. According to an embodiment of the present invention, the tag generation unit 120 may generate tag information relating to an ultrasound image acquired during an ultrasonic diagnosis in real time or may generate tag information relating to a previously stored ultrasound image. According to an embodiment of the present invention, the tag generation unit 120 may generate a plurality of pieces of information input by a user relating to an ultrasound image as tag information.
The tag information according to the present embodiment may include measurement information, annotation information, indication information, body mark information, and the like.

The measurement information means information regarding a physical measurement result acquired through the ultrasonic diagnosis. Examples of the measurement information may include a bi-partial diameter (BPD), an occipitofrontal diameter (OFD), an abdominal circumference (AC), a femur length (FL), and the like.

The annotation information means information in which content analyzed by the user (a doctor, etc.), a user's opinion on abnormality, and a singularity, and the like regarding the ultrasound image are recorded.

The indication information means information regarding a specific part of the ultrasound image indicated by the user (the doctor, a medical laboratory technologist, etc.) indication may be expressed in various ways including an arrow, a finger shape, etc. According to an embodiment of the present invention, the user indicates specific parts using various colors to generate the indication information.

A body mark means a diagram presenting a location or a target to which ultrasonic waves are scanned. The body mark may be, for example, a shape of liver, etc.

Meanwhile, the tag information may include an icon indicating a type of a tag, etc. For example, the tag information may include an icon

" ⌊_⌊_⌊_⌊_⌊_⌋ "

indicating the measurement information, an icon "①" indicating the annotation information, an icon "①" indicating the indication information, and the like.

The storage unit 130 may store patient information, ultrasound image data, etc. According to an embodiment of the present invention, the storage unit 130 may be embedded in the ultrasound image data providing apparatus 100 or may be an external memory. The storage unit 130 also may be a storage server implemented on the Web.

The storage unit 130 according to the present embodiment may classify and store the ultrasound image data, according to patients and diagnosis dates. According to an embodiment of the present invention, the storage unit 130 may store ultrasound images in a unit of the examination items EXAM. For example, if the user measures the BFD, the OFD, the AC, and the FL at a predetermined time interval, ultrasound images of the measured BFD, OFD, AC, and FL may form one EXAM.

The patient information of the present embodiment may include information regarding patient personal identification information (an ID, an ID number, a name, a birth date, and the like), a medical history, a singularity, and the like.

The ultrasound image data of the present embodiment may include the ultrasound image and the tag information. The ultrasound image data may include a plurality of ultrasound images and a plurality of pieces of tag information each corresponding to the ultrasound images. Meanwhile, the storage unit 130 may store a comparison result with respect to the ultrasound images.

The receiving unit 140 may receive a predetermined search word. According to an embodiment of the present invention, the receiving unit 140 may receive a predetermined search word that the user personally inputs, and may receive a predetermined search word that the user selects on a displayed screen.

The search word according to the present embodiment may include text including a character or a number, etc. According to another embodiment of the present invention, the search word may be a predetermined image. For example, the search word may include a specific image including a shape (for example, the arrow or the shape of finger, etc.) of the indication, the body mark (liver, heart, womb, fetus, etc.), and the like.

According to an embodiment of the present invention, the receiving unit 140 may receive selection information regarding a specific tag included in the ultrasound image data displayed on a screen. For example, the user may select the BPD, the OFD, the body mark, etc. from the screen on which the ultrasound image data is displayed.

The receiving unit 140 may receive the annotation information regarding a measurement result, a diagnosis comment, a singularity, etc. from the user during the ultrasonic diagnosis. For example, the receiving unit 140 may receive additional tag information relating to the displayed ultrasound image data from the user. The additional tag information may mean the measurement information, the annotation information, the indication information, the body mark information, and the like that are additionally received from the user, besides basic tag information included in the ultrasound image data.

The extraction unit 150 may extract the ultrasound image data including tag information related to the predetermined search word. In this case, according to an embodiment of the present invention, the extraction unit 150 may extract a plurality of pieces of ultrasound image data including the information related to the predetermined search word.

The display unit 160 may display information processed by the ultrasound image data providing apparatus 100. For example, the display unit 160 may display the ultrasound image data. That is, the display unit 160 may display the ultrasound image and the tag information. In this case, the display unit 160 may display a plurality of pieces of tag information corresponding to the ultrasound image.

Meanwhile, according to an embodiment of the present invention, the display unit 160 may display the ultrasound image data extracted by the extraction unit 150. If the extraction unit 150 extracts a plurality of ultrasound image data, the display unit 160 may display a plurality of ultrasound images and a plurality of pieces of tag information each corresponding thereto.

If the display unit 160 and a touch pad form a mutual layer structure and thus are configured as a touch screen, the display unit 160 may be used as an input device other than an output device. The display unit 160 may include at least one of a liquid crystal display (LCD), a thin film transistor-liquid crystal display (TFT-LCD), an organic light-emitting diode (OLED), a flexible display, and a 3D display. The ultrasound image data providing apparatus 100 may also include two or more display units 160. The touch screen may be configured to detect a touch input pressure as well as a touch input location and a touched area. The touch screen may also be configured to detect a proximity touch as well as a real touch.

The display unit 160 according to the present embodiment may display a result obtained by comparing the extracted plurality of pieces of ultrasound image data. In this case, the display unit 160 may visually display the comparison result in a graph or in a chart.

Meanwhile, if the user selects at least one tag, the display unit 160 may display an ultrasound image corresponding to the selected at least one tag.

If an ultrasound image is acquired, the display unit 160 may also provide the acquired ultrasound image and tag information related to the ultrasound image together. For example, the display unit 160 may display the ultrasound image and measurement information, annotation information, indication information, and body mark information related to the ultrasound image together.

If the additional tag information related to the displayed ultrasound image data is input, the display unit 160 may display the ultrasound image data including the additional tag information.

The control unit 170 may generate the result obtained by comparing the extracted plurality of pieces of ultrasound image data as tag information. The control unit 170 may also control the storage unit 130 to store ultrasound image data including the tag information generated based on the comparison result.

That is, the control unit 170 may generate new ultrasound image data including the tag information generated based on the comparison result, the plurality of pieces of ultrasound image data that is a comparison target, and tag information corresponding to the plurality of pieces of ultrasound image data that is the comparison target and control the storage unit 130 to store the new ultrasound image data.

The control unit 170 may generally control the acquisition unit 110, the tag generation unit 120, the storage unit 130, the receiving unit 140, the extraction unit 150, and the display unit 160.

A method of efficiently providing ultrasound image data by using each element of the ultrasound image data providing apparatus 100 will now be in detail described with reference to FIGS. 2 through 4.

FIG. 2 is a flowchart illustrating an ultrasound image data providing method, according to an embodiment of the present invention.

Referring to FIG. 2, according to the present embodiment, the ultrasound image data providing apparatus 100 may store ultrasound image data including ultrasound images and tag information relating to the ultrasound images (S210).

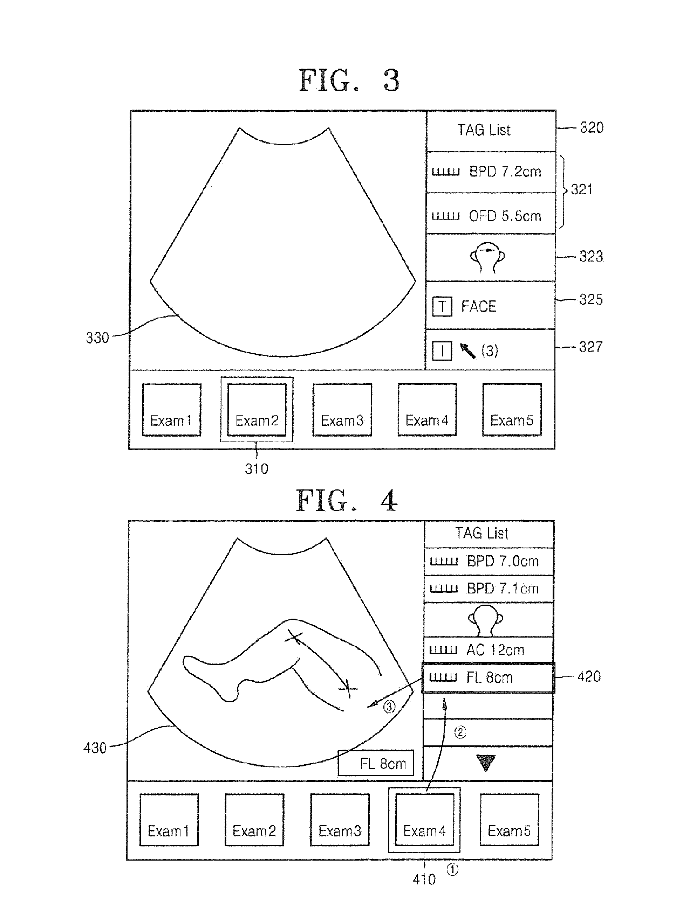

For example, as shown in FIG. 3, the ultrasound image data providing apparatus 100 may store an ultrasound image 330 and tag information 320 in a unit of examination EXAM 310. If a circumference of a fetus's head is measured, the tag information 320 may include measurement information 321 such as BPD, OFD, etc. body mark information 323 indicting the circumference of the fetus's head, annotation information 325 such as a face input as text, indication information 327 including a shape of indication (for example, an arrow), the number of indications (for example, 3), etc.

According to an embodiment of the present invention, the annotation information 325 that a user inputs on a displayed screen may be displayed in real time, and may be stored as the tag information 320 regarding a corresponding EXAM later.

The ultrasound image data providing apparatus 100 may receive a predetermined search word (S220). For example, the user may input a search word such as "OFD, BPD > 7cm, shapes of indications (e.g., ▼, ↑, ✎ ), number of indications: 3" or may select a specific body mark.

The ultrasound image data providing apparatus 100 may extract ultrasound image data including tag information relating to the predetermined search word (S230). According to an embodiment of the present invention, the extracted ultrasound image data may include an ultrasound image and tag information regarding a predetermined examination EXAM.

For example, if a search word "OFD" is received, the ultrasound image data providing apparatus 100 may extract ultrasound image data including an OFD measurement value as tag information. If a search word "three indications" is input, the ultrasound image data providing apparatus 100 may also extract ultrasound image data including three indications. Meanwhile, if the user selects a "head" from body marks, the ultrasound image data providing apparatus 100 may also extract ultrasound image data including tag information regarding the head.

The ultrasound image data providing apparatus 100 may display the extracted ultrasound image data (S240). That is, the ultrasound image data providing apparatus 100 may display tag information relating to a search word and an ultrasound image corresponding to the tag information. In this regard, the ultrasound image data providing apparatus 100 may display a plurality of pieces of tag information and ultrasound images relating to the search word.

Meanwhile, the ultrasound image data providing apparatus 100 may receive selection information regarding at least one tag included in the displayed ultrasound image data. In this case, the ultrasound image data providing apparatus 100 may display an ultrasound image corresponding to the at least one tag.

For example, as shown in FIG. 4, in a case where ultrasound image data regarding EXAM 4 410 is extracted and displayed (operation ①), the user may select at least one tag from a displayed tag list. In a case where the user selects "FL" 420 that is measurement information from the tag list (operation ②), the ultrasound image data providing apparatus 100 may display an ultrasound image 430 relating to the "FL" 420 (operation ③).

In a case where the user selects "BPD 7cm" from the tag list, the ultrasound image data providing apparatus 100 may display an ultrasound image relating to the BPD 7cm".

Meanwhile, the ultrasound image data providing apparatus 100 may receive additional tag information relating to the displayed ultrasound image data from the user, and display the ultrasound image data including the input additional tag information.

Thus, according to an embodiment of the present invention, the user may use tag information to efficiently search for ultrasound image data. The ultrasound image data providing apparatus 100 may provide the user with an ultrasound image relating to each tag selected by the user in real time.

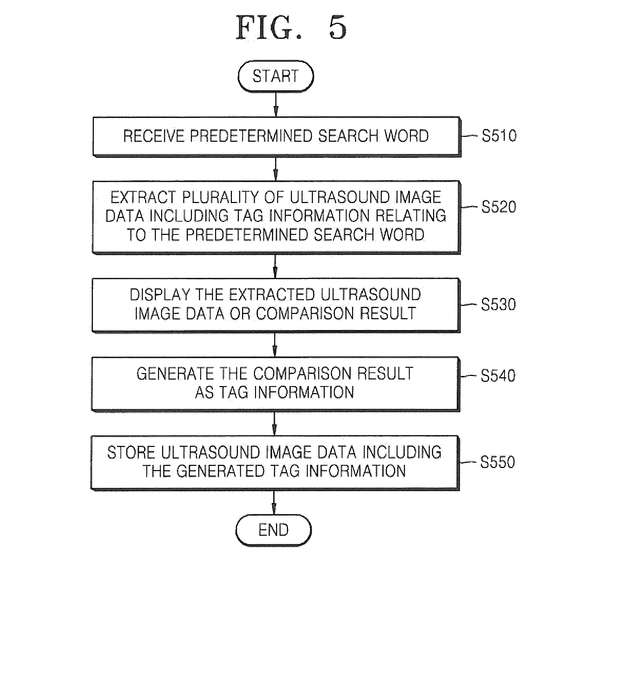

A method of extracting a plurality of pieces of ultrasound image data and a method of providing comparison data by using the ultrasound image data providing apparatus 100 will now be in detail described with reference to FIGS. 5 through 7.

FIG. 5 is a flowchart illustrating a method of providing a plurality of pieces of ultrasound image data, according to an embodiment of the present invention.

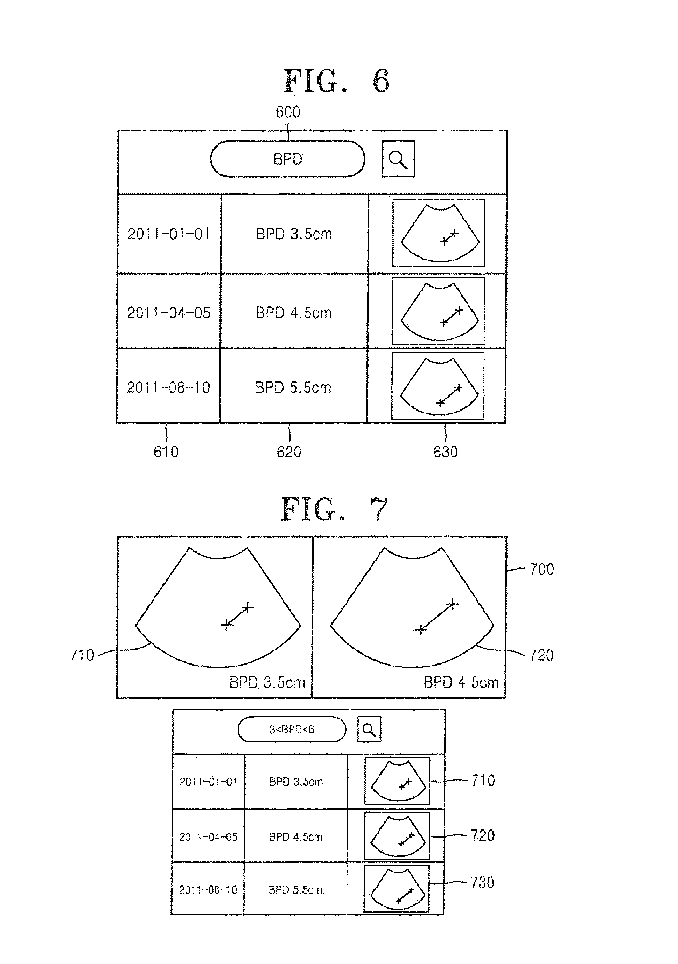

Referring to FIG. 5, according to the present embodiment, the ultrasound image data providing apparatus 100 may receive a predetermined search word (S510). For example, the ultrasound image data providing apparatus 100, as shown in FIG. 6, may receive a search word "BPD" 600. The ultrasound image data providing apparatus 100, as shown in FIG. 7, may also receive a measurement value range such as "3 < BPD < 6" as the search word.

In this case, the ultrasound image data providing apparatus 100 may extract a plurality of pieces of ultrasound image data including tag information relating to the predetermined search word (S520). For example, as shown in FIG. 6, if the search word "BPD" 600 is input, the ultrasound image data providing apparatus 100 may extract a plurality of pieces of ultrasound image data including tag information relating to the "BPD" 600.

The ultrasound image data providing apparatus 100 may display the extracted plurality of pieces of ultrasound image data (S530). According to an embodiment of the present invention, the ultrasound image data providing apparatus 100 may display the extracted plurality of pieces of ultrasound image data in a list format. For example, as shown in FIG. 6, a list of plurality of pieces of ultrasound image data may include a BPD measurement date 610, a BPD value 620, a BPD related ultrasound image 630, and the like.

According to an embodiment of the present invention, the ultrasound image data providing apparatus 100 may display a result obtained by comparing the extracted plurality of pieces of ultrasound image data (S530).

For example, as shown FIG. 7, in a case where the user inputs the search word "3 < BPD < 6", the ultrasound image data providing apparatus 100 may display ultrasound image data relating to the "3 < BPD < 6". In this case, the user may select specific ultrasound image data of which detailed comparison is desired from the displayed plurality of pieces of ultrasound image data. In a case where the user selects ultrasound image data 710 having BPD 3.5cm and ultrasound image data 720 having BPD 4.5cm, the ultrasound image data providing apparatus 100 may compare and display the two ultrasound image data 710 and 720 selected by the user.

Meanwhile, according to an embodiment of the present invention, the ultrasound image data providing apparatus 100 may visually display the comparison result with respect to the extracted plurality of pieces of ultrasound image data in a graph or in a chart. According to an embodiment of the present invention, the user may compare and diagnose ultrasound image data searched by using tag on division screens, thereby efficiently observing a progress of a specific part.

The ultrasound image data providing apparatus 100 may generate the comparison result as tag information (S540). In this case, the ultrasound image data providing apparatus 100 may generate and store new ultrasound image data including the tag information (S550). According to an embodiment of the present invention, the new ultrasound image data may include ultrasound images and tag information included in the extracted plurality of pieces of ultrasound image data.

According to an embodiment of the present invention, the new ultrasound image data inducing the generate tag information may be review data with respect to original ultrasound image data.

According to an embodiment of the present invention, the ultrasound image data providing apparatus 100 may display ultrasound image data relating to a predetermined search word input by a user, and thus the user may reduce time taken to search for the ultrasound image data, and efficiently utilize the ultrasound image data.

According to an embodiment of the present invention, tag information relating to ultrasound images is displayed together with the ultrasound images, and thus the user may easily check the whole information regarding the ultrasound images.

Meanwhile, the user may find and review a previously stored ultrasound image by using a user's opinion on abnormality or a specific value that occurs during measurement without depending on an image thumbnail.

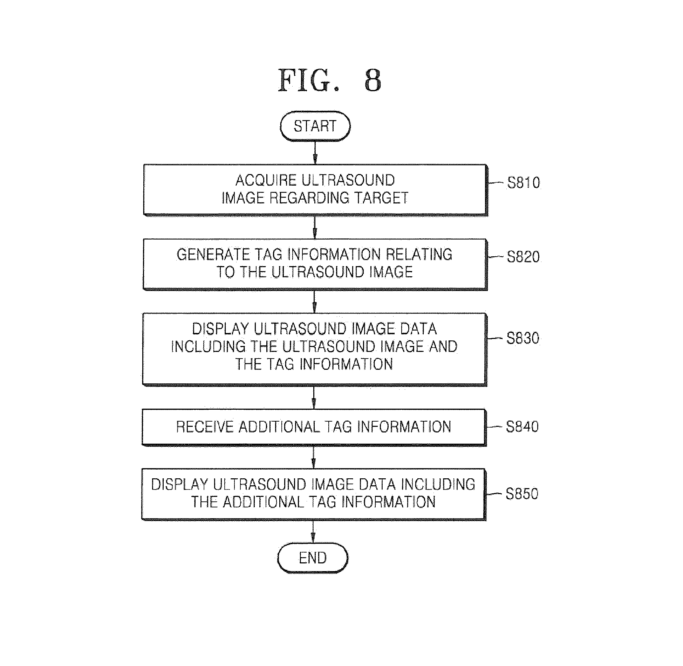

FIG. 8 is a flowchart illustrating a method of displaying ultrasound image data, according to an embodiment of the present invention.

Referring to FIG. 8, according to the present embodiment, the ultrasound image data providing apparatus 100 may acquire an ultrasound image regarding a target (S810). In this regard, the ultrasound image data providing apparatus 100 may generate tag information relating to the ultrasound image (S820). According to an embodiment of the present invention, the ultrasound image data providing apparatus 100 may extract measurement information, indication information, etc. from the acquired ultrasound image and generate the tag information. According to another embodiment of the present invention, the ultrasound image data providing apparatus 100 may receive annotation information, body mark information, etc. from a user and generate the tag information.

According to the present embodiment, the ultrasound image data providing apparatus 100 may display ultrasound image data including the ultrasound image and the tag information (S830). That is, the ultrasound image data providing apparatus 100 may display the acquired ultrasound image and a plurality of pieces of tag information corresponding to the acquired ultrasound image to the user.

Thus, according to an embodiment of the present invention, the user may simultaneously check the ultrasound image and the tag information relating to the ultrasound image by using the ultrasound image data providing apparatus 100, which enhances convenience of ultrasonic diagnosis.

The ultrasound image data providing apparatus 100 may receive additional tag information (S840). In this regard, the ultrasound image data providing apparatus 100 may ultrasound image data including the additional tag information (S850).

That is, if additional information (for example, annotation information, indication information, measurement information, and body mark information) regarding the displayed ultrasound image is input, the ultrasound image data providing apparatus 100 may generate the input additional information as a tag and display the tag in real time.

Embodiments of the present invention included a computer-readable recording medium including program commands for executing operations implemented through various computers. The computer-readable recording medium can store program commands, data files, data structures, or combinations thereof. The program commands recorded in the computer-readable recording medium may be specially designed and configured for the present invention or be known to those of ordinary skill in the field of computer software. Examples of a computer-readable recording medium include magnetic media such as hard disks, floppy disks, and magnetic tapes, optical media such as CD-ROMs and DVDs, magneto-optical media such as floptical disks, or hardware devices such as ROMs, RAMs, and flash memories, which are specially configured to store and execute program commands. Examples of the program commands include a machine language code created by a compiler and a high-level language code executable by a computer using an interpreter and the like. The computers may include the control unit 170 of the ultrasound image data providing apparatus 100.

While the present invention has been particularly shown and described with reference to exemplary embodiments thereof, it will be understood by those of ordinary skill in the art that various changes in form and details may be made therein without departing from the spirit and scope of the present invention as defined by the following claims.

**Claims**

1. An ultrasound image data providing method, the method comprising:

   storing ultrasound image data including an ultrasound image and tag information relating to the ultrasound image;
   receiving a predetermined search word;
   extracting ultrasound image data including tag information relating to the predetermined search word; and
   displaying the extracted ultrasound image data.

2. The method of claim 1, wherein the tag information comprises at least one of measurement information, annotation information, indication information, and body mark information.

3. The method of claim 1, wherein the predetermined search word comprises at least one of text and an image.

4. The method of claim 1, wherein the displaying comprises:

   receiving selection information regarding at least one tag included in the displayed ultrasound image data; and
   displaying an ultrasound image corresponding to the at least one tag selected according to the selection information.

5. The method of claim 1, wherein the displaying comprises:

   receiving additional tag information relating to the displayed ultrasound image data from a user; and
   displaying the ultrasound image data including the received additional tag information.

6.  The method of claim 1, wherein the extracting comprises: extracting a plurality of pieces of ultrasound image data including the tag information relating to the predetermined search word,
    wherein the displaying comprises: displaying the extracted plurality of pieces of ultrasound image data.

7.  The method of claim 6, wherein the displaying further comprises: displaying a result obtained by comparing the extracted plurality of pieces of ultrasound image data.

8.  The method of claim 7, wherein the storing ultrasound image data comprises:

    generating a comparison result as tag information; and
    storing ultrasound image data including the generated tag information.

9.  An ultrasound image data providing method, the method comprising:

    acquiring an ultrasound image regarding a target;
    generating tag information relating to the acquired ultrasound image; and
    displaying ultrasound image data including the acquired ultrasound image and the generated tag information.

10. The method of claim 9, wherein the tag information comprises at least one of measurement information, annotation information, indication information, and body mark information.

11. The method of claim 9, wherein the displaying of the ultrasound image data comprises:

    receiving additional tag information relating to the displayed ultrasound image data; and
    displaying the ultrasound image data including the received additional tag information.

12. An ultrasound image data providing apparatus, the apparatus comprising:

    a storage unit for storing ultrasound image data including an ultrasound image and tag information relating to the ultrasound image;
    a receiving unit for receiving a predetermined search word;
    an extraction unit for extracting ultrasound image data including tag information relating to the predetermined search word;
    a displaying unit for displaying the extracted ultrasound image data; and
    a control unit for controlling the storage unit, the receiving unit, the extraction unit, and the display unit.

13. The apparatus of claim 12, wherein the extraction unit extracts a plurality of pieces of ultrasound image data including the tag information relating to the predetermined search word,
    wherein the display unit displays the extracted plurality of pieces of ultrasound image data.

14. An ultrasound image data providing apparatus, the apparatus comprising:

    an acquisition unit for acquiring an ultrasound image regarding a target;
    a tag generation unit for generating tag information relating to the acquired ultrasound image; and
    a display unit for displaying ultrasound image data including the acquired ultrasound image and the generated tag information.

15. A computer readable recording medium storing a program for executing an ultrasound image data providing method of claim 1.

# FIG. 1

100

**ULTRASOUND IMAGE DATA PROVIDING APPARATUS**

130
STORAGE UNIT

140
RECEIVING UNIT

120
TAG GENERATION UNIT

170
CONTROL UNIT

150
EXTRACTION UNIT

110
ACQUISITION UNIT

160
DISPLAY UNIT

# FIG. 2

START

STORE ULTRASOUND IMAGE DATA INCLUDING ULTRASOUND IMAGES AND TAG INFORMATION — S210

RECEIVE PREDETERMINED SEARCH WORD — S220

EXTRACT ULTRASOUND IMAGE DATA INCLUDING TAG INFORMATION RELATING TO THE PREDETERMINED SEARCH WORD — S230

DISPLAY THE EXTRACTED ULTRASOUND IMAGE DATA — S240

END

# FIG. 3

# FIG. 4

# FIG. 5

START

RECEIVE PREDETERMINED SEARCH WORD ── S510

EXTRACT PLURALITY OF ULTRASOUND IMAGE DATA INCLUDING TAG INFORMATION RELATING TO THE PREDETERMINED SEARCH WORD ── S520

DISPLAY THE EXTRACTED ULTRASOUND IMAGE DATA OR COMPARISON RESULT ── S530

GENERATE THE COMPARISON RESULT AS TAG INFORMATION ── S540

STORE ULTRASOUND IMAGE DATA INCLUDING THE GENERATED TAG INFORMATION ── S550

END

# FIG. 6

# FIG. 7

# FIG. 8

START

ACQUIRE ULTRASOUND
IMAGE REGARDING TARGET —— S810

GENERATE TAG INFORMATION RELATING
TO THE ULTRASOUND IMAGE —— S820

DISPLAY ULTRASOUND IMAGE DATA
INCLUDING THE ULTRASOUND IMAGE AND
THE TAG INFORMATION —— S830

RECEIVE ADDITIONAL TAG INFORMATION —— S840

DISPLAY ULTRASOUND IMAGE DATA INCLUDING
THE ADDITIONAL TAG INFORMATION —— S850

END

**EP 2 624 155 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120011794